# EUROPEAN PATENT SPECIFICATION

(11) **EP 3 052 045 B1**
(45) Date of publication and mention of the grant of the patent: **19.07.2017**
(21) Application number: 14790363.7
(22) Date of filing: 05.09.2014
(51) Int. Cl.: A61C 7/02, A61C 7/10

(54) **TOOL FOR THE ACTIVATION OF PALATAL EXPANDERS**
WERKZEUG ZUR AKTIVIERUNG VON GAUMENEXPANDERN
OUTIL POUR L'ACTIVATION D'ÉCARTEURS PALATINS

(30) Priority: 01.10.2013 IT FI20130227
(43) Date of publication of application: 10.08.2016
(73) Proprietor: Leone S.p.A., 50019 Sesto Fiorentino (Firenze) (IT)
(72) Inventor: DOLFI, Maurizio, 50143 Firenze (IT); SCOMMEGNA, Gabriele, 50029 Tavarnuzze Impruneta (FI) (IT)
(74) Representative: Mincone, Antimo
(86) International application number: PCT/IT2014/000236
(87) International publication number: WO 2015/049706

(56) References cited:
- US-A- 5 133 659
- US-A1- 2003 013 061
- US-B1- 6 174 162

## Description

The present invention relates to a tool, in jargon called "key", for the activation of palatal expanders. Keys for palatal expanders are known for example from US2003/0013061 and US6174162. It is known that the palatal expanders are used in the treatment of deficiency of the maxillary arch that require the disjunction of the palatal suture. Such expanders are generally constituted by two bodies slidably mounted on parallel guide rods and constrained, by means of shaped arms, to a predetermined number of teeth of the dental arch to be treated. Said bodies are also threaded to receive, each, a stem of a screw having a double threadening through which the same bodies can be moved away from each other thereby causing the effect of expansion desired. Said screw has a maneuvering or operating head resulting in a central position between said two bodies. The operating head of the screw has a plurality of holes destined to be engaged by a tool, commonly called "key", allowing the screw to be rotated at predetermined time intervals according to a therapeutic program indicated by the dentist. Usually, the therapeutic program provides for the rotation of the screw of a quarter of a turn at regular time intervals, for example a quarter of a turn once a day for ten days, depending on the type and severity of the deficiency to be corrected. The periodic operation of the screw is generally entrusted to the relatives of the patient.

A problem encountered in the use of the screw-operated expanders described above lies in that, especially in the case of treatments particularly prolonged in time, the user does not keep properly track of the number of turns to which the screw has been subjected, and then of the degree of expansion reached, which can be harmful for the patient and may compromise the effectiveness of the treatment. The main purpose of the present invention is to eliminate, or at least greatly reduce, the drawbacks mentioned above.

This result is achieved, according to the present invention, by providing a device having the characteristics indicated in claim 1. Other features of the present invention are the subject of the dependent claims.

Thanks to the present invention, it is possible to easily keep track of the number of turns or fraction of turns of the screw, without making use of external means, greatly reducing the risk of compromising the efficacy of the treatment. Furthermore, a tool in accordance with the present invention is simple to be made and is inexpensive in relation to the advantages offered. These and other advantages and features of the present invention will be best understood by anyone skilled in the art thanks to the following description and the accompanying drawings, given by way of example but not to be considered in a limiting sense, wherein:
- Fig.1 is a perspective view of a tool in accordance with the present invention;

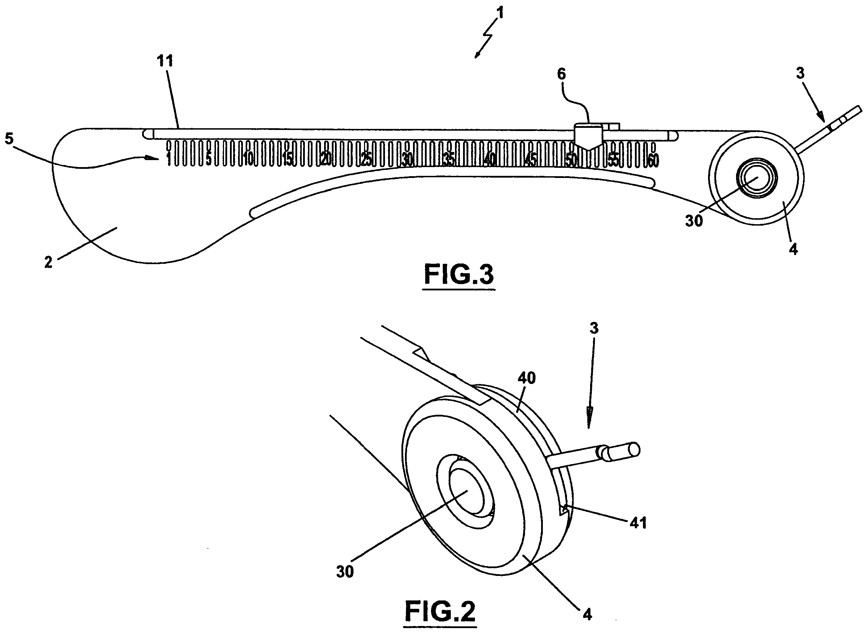

- Fig.2 is an enlarged detail of the tool shown in Fig.1;
- Fig.3 is a side view of the tool shown in Fig.1;

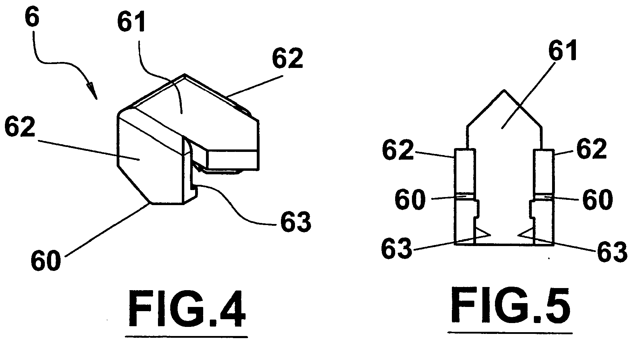

- Fig.4 is a perspective view of the slider (6);
- Fig. 5 is a bottom view of the slider shown in Fig.4;

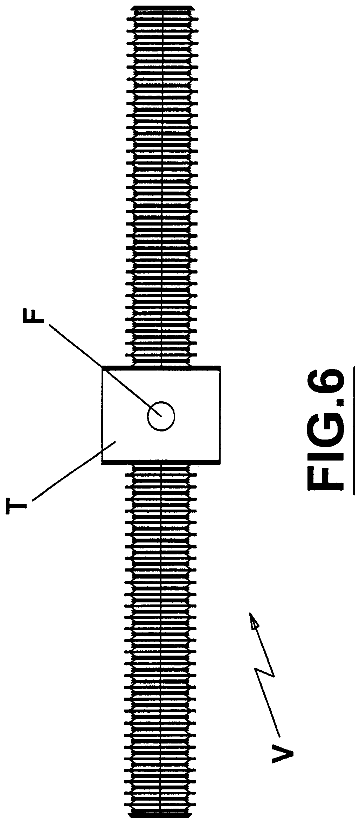

- Fig.6 schematically shows a screw for palatal expanders of the known type;
- Fig.7 schematically shows a screw-operated palatal expander of the known type;

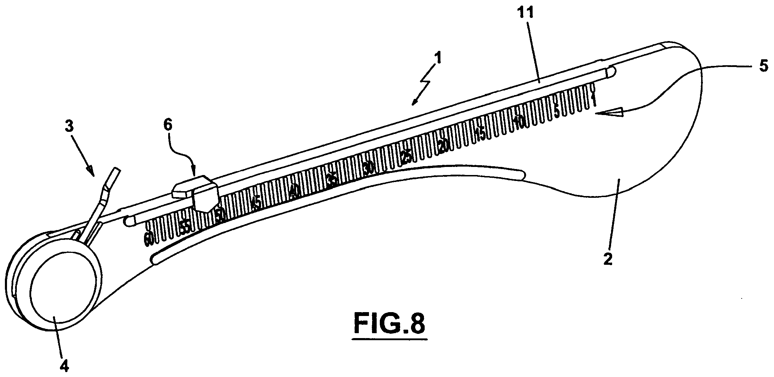

- Fig.8 is another perspective view of the tool shown in Fig.1;
- Fig.9 shows an enlarged detail of Fig.8.

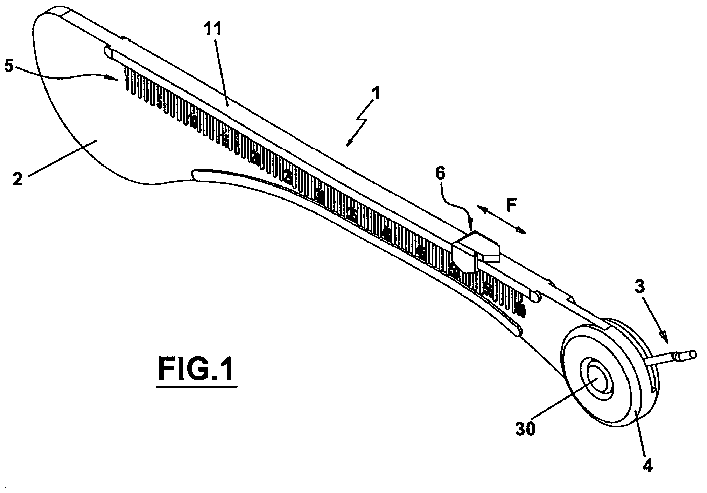

Reduced to its basic structure and with reference to the attached drawings, a tool for the activation of a screw-operated palatal expander in accordance with the present invention comprises a body (1) having predominant longitudinal development, i.e. having a length that is greater than the height and thickness, which has a grip portion (2) and, on the opposite side, a tip (3). In this example, the tip (3) is applied, by means of a pin (30) with axis orthogonal to the two sides (10) of the body (1), in a capsule (4) which has a slot (40) within which the same tip (3) can rotate around the axis of the pin (30). The said slot (40) has an abutment point (41) which limits the angular movement of the tip (3) such that the tip (3) can rotate up to interfering with the abutment point. This basic structure of the tool is known per se. Advantageously, in accordance with the present invention, the body (1) has, along at least one of its sides (10), a series of marks (5) and, above said marks (5), a slider (6) that is provided with an appendix (60) facing the same marks (5). The slider (6) is slidingly applied on an upper edge (11) of the body (1) above the marks (5) so that, by sliding along said edge (11) of the body (1), as indicated by arrow "F" in Fig.1 the aforementioned appendix (60) can be positioned in correspondence of a mark (5).

The marks (5) can be shaped in any way. For example, the marks (5) can be numbers and/or notches provided on one side of the body (1) or on both the left and right sides of the latter, aligned along a direction parallel to that of the edge (11).

The slider (6) is slightly forced on the edge (11) of the body (1) to prevent it from sliding spontaneously.

The slider (6), in accordance with the example shown in the drawings, has an upper surface (61) and two side surfaces (62), the latter being orthogonal to the upper surface (61). The inner side of the upper surface (61) is turned towards the upper edge (11) of the body (1). The side surfaces (62) have each an internal tooth (63) which engages the lower side of the upper edge (11) of the body (1) that, for this purpose, protrudes from both sides of the same body (1). Furthermore, the side surfaces (62) have each two sides converging towards the bottom that define the aforementioned appendix (60). The upper surface (61) is advantageously shaped so as to exhibit a pointed front part that indicates the direction along which the slider (6) must be made to slide. Each time the tool is used (by inserting the tip 3 into a hole F of the operating head T of the screw V of a screw-operated palatal expander E), the slider (6) is manually advanced along the edge (11) of the body (1), the run of the slider corresponding to the distance between two adjacent marks. In this way, the appendix (60) of the slider (6) moves on the marks (5) recording each time the use of the tool, i.e. the activation of the expander. Therefore, the user is facilitated in keeping track of the number of activations run without external tracking as the tracking mechanism is integrated with the tool.

In practice, a tool in accordance with the present invention is provided with means for displaying the number of uses.

According to the example described above, the means for displaying the number of uses comprise the marks (5) and the slider (6) and the latter has an indicator (60) in correspondence of the marks (5). It goes without saying, however, that the means for displaying the number of uses may be structured differently.

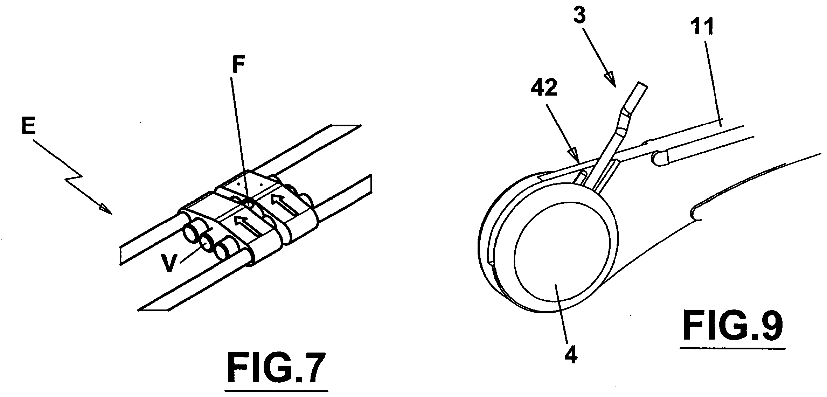

As shown in Fig.9, the slot (40) is provided with a protuberance (42) that, when the tip (3) passes in front of it during the activation of the expander, produces a "click" sound alerting the user that the tip (3) has completed a run, and then that the screw (V) has been rotated and consequently the expander (E) has been activated.

In practice the details of execution may vary in any equivalent way as for what concerns the individual elements described and illustrated and to their arrangement without departing from the scope of the adopted solution as defined by the set of claims.

## Claims

1. Tool for actuating palatal expanders, comprising a body (1) with a grip portion (2) and, on the opposite side, a tip (3), **characterized in that** the said body (1) has, on at least one surface(10) thereof, display means adapted for displaying the number of uses.

2. Tool according to claim 1 **characterized in that** said display means comprise marks (5) on at least one side surface (10) of the body (1) and a slider (6) slidably applied on the same body (1), said slider (6) having an indicator (60) that can be moved on said indices (5).

3. Tool according to claim 2 **characterized in that** the said marks (5) consist of numbers and/or notches.

4. Tool according to claim 2 **characterized in that** the said slider (6) is applied on an upper edge (11) of the body (1).

5. Tool according to claim 2 **characterized in that** said slider (6) has an upper surface (61) and two side surfaces (62) orthogonal to the upper surface (61), **in that** an inner side of the upper surface (61) is turned towards an upper edge (11) of the body (1), **in that** the said side surfaces (62) have each an internal tooth (63) which engages the lower side of the upper edge (11) of the body (1), and **in that** the upper edge (11) of the body (1) protrudes from both sides beyond at least one side of the same body (1).

6. A tool according to claim 1, **characterized in that** the said tip (3) is applied, by means of a pin (30) with axis orthogonal to two lateral surfaces (10) of the body (1), in a capsule (4) having a slot (40) within which the same tip (3) can rotate around the axis of the pin (30).

7. Tool according to claim 6 **characterized in that** said slot (40) has an abutment point (41) that limits the angular movement of the tip (3).

8. Tool according to claim 6 **characterized in that** said slot (40) has an appendix (42) which interferes with the tip (3) when the angular run of the latter is completed thus producing the sound of a click.

## Patentansprüche

1. Werkzeug zur Betätigung von Gaumenexpandern, das einen Körper (1) mit einem Griffteil (2) und einer auf der gegenüberliegenden Seite befindlichen Spitze (3) umfasst, **dadurch gekennzeichnet, dass** der Körper (1) auf wenigstens einer Oberfläche (10) Anzeigeeinrichtungen aufweist, die dazu dienen, die Anzahl der Verwendungen anzuzeigen.

2. Werkzeug nach Anspruch 1, **dadurch gekennzeichnet, dass** die Anzeigeeinrichtungen Markierungen (5) auf wenigstens einer Seitenoberfläche (10) des Körpers (1) und einen Schlitten (6) umfassen, der an dem Körper (1) in verschiebbarer Weise montiert ist, wobei der Schlitten (6) einen Indikator (60) besitzt, der auf den Indexmarkierungen (5) bewegt werden kann.

3. Werkzeug nach Anspruch 2, **dadurch gekennzeichnet, dass** die Markierungen (5) aus Ziffern und/oder Kerben bestehen.

4. Werkzeug nach Anspruch 2, **dadurch gekennzeichnet, dass** der Schlitten (6) an einer oberen Kante (11) des Körpers (1) angeordnet ist.

5. Werkzeug nach Anspruch 2, **dadurch gekennzeichnet, dass** der Schlitten (6) eine obere Oberfläche (61) und zwei senkrecht zur oberen Oberfläche (61) verlaufende Seitenflächen (62) besitzt, dass die Innenseite der oberen Oberfläche (61) zu einer oberen Kante (11) des Körpers (1) zugewandt ist, dass die Seitenoberflächen (62) jeweils einen internen Zahn (63) aufweisen, der mit der unteren Seite der oberen Kante (11) des Körpers (1) in Eingriff steht, und dass die obere Kante (11) des Körpers (1) von beiden Seiten über wenigstens eine Seite des Körpers (1) hinaus vorsteht.

6. Werkzeug nach Anspruch 1, **dadurch gekennzeichnet, dass** besagte Spitze (3) vermittels eines Stiftes (30), dessen Achse senkrecht zu den beiden Seitenflächen (10) des Körpers (1) verläuft, in einer Kapsel (4) angeordnet ist, die einen Schlitz (40) besitzt, in welchem sich die Spitze (3) um die Achse des Stiftes (30) drehen kann.

7. Werkzeug nach Anspruch 6, **dadurch gekennzeichnet, dass** der Schlitz (40) einen Anschlagspunkt (41) aufweist, der die Winkelbewegung der Spitze (3) begrenzt.

8. Werkzeug nach Anspruch 6, **dadurch gekennzeichnet, dass** der Schlitz (40) einen Fortsatz (42) besitzt, der mit der Spitze (3) in Eingriff tritt, wenn deren Winkelbewegung vervollständigt ist und dabei einen Klickton erzeugt.

## Revendications

1. Outil pour l'activation d'écarteurs palatins, comprenant un corps (1) essentiellement à développement longitudinal, c'est-à-dire avec une dimension prédominante par rapport aux deux autres, lequel présente une portion de prise (2) et, d'un côte opposé, une pointe (3) **caractérisée en ce que** ledit corps (1) présente, sur au moins une des ces surfaces (10), des moyens de visualisation du nombre d'utilisations.

2. Outil selon la revendication 1 **caractérisé en ce que** lesdits moyens de visualisation comprennent des indices (5) sur au moins une face ou superficie latérale (10) du corps (1) et un curseur (6) appliqué coulissant sur le corps même (1), ledit curseur (6) présentant un indicateur (60) en correspondance desdits indices (5).

3. Outil selon la revendication 2 **caractérisé en ce que** lesdits indices (5) sont constitués par des chiffres et/ou des crans.

4. Outil selon la revendication 2 **caractérisé en ce que** ledit curseur (6) est appliqué sur un bord supérieur (11) du corps (1).

5. Outil selon la revendication 2 **caractérisé en ce que** ledit curseur (6) présente une surface supérieure (61) et deux surfaces latérales (62) orthogonales à la surface supérieure (61), **en ce que** une face interne de la surface supérieure (61) est tournée vers le bords supérieur (11) du corps (1), **en ce que** lesdites surfaces latérales (62) présentent chacune un dent interne (63) qui engage le versant inférieur du bord supérieur (11) du corps (1), et **en ce que** le bord supérieur (11) du corps (1) est proéminent de chaque côté au-delà les faces ou les surfaces latérales du corps (1) même.

6. Outil selon la revendication 1 **caractérisé en ce que** ladite pointe (3) est appliquée, au moyens d'un pivot (30) avec axe orthogonal à deux surface latérales (10) du corps (1), dans une capsule (4) qui présente une boutonnière à l'intérieur de laquelle la pointe même (3) peut tourner autour de l'axe du pivot (30).

7. Outil selon la revendication 6 **caractérisé en ce que** ladite boutonnière (40) présente un point de butée (41) qui limite la course totale angulaire de la pointe (3).

8. Outil selon la revendication 6 **caractérisé en ce que** ladite boutonnière (40) présente un appendice (42) avec lequel la pointe (3) entre en contact en produisant le bruit d'un déclenchement lorsque sa course totale est complétée.
